(19) Europäisches Patentamt
European Patent Office
Office européen des brevets

(11) **EP 4 516 122 A1**

(12) **EUROPEAN PATENT APPLICATION**
published in accordance with Art. 153(4) EPC

(43) Date of publication:
**05.03.2025 Bulletin 2025/10**

(21) Application number: **23796542.1**

(22) Date of filing: **28.04.2023**

(51) International Patent Classification (IPC):
***A24B 13/00*** *(2006.01)*

(52) Cooperative Patent Classification (CPC):
**A24B 13/00**

(86) International application number:
**PCT/JP2023/016862**

(87) International publication number:
**WO 2023/210810 (02.11.2023 Gazette 2023/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.04.2022 JP 2022074829**

(71) Applicant: **Japan Tobacco Inc.
Tokyo 105-6927 (JP)**

(72) Inventors:
• **FURUKOSHI, Masashi
Tokyo 130-8603 (JP)**

• **MIYAUCHI, Masato
Tokyo 130-8603 (JP)**
• **MUTO, Hiromichi
Tokyo 130-8603 (JP)**
• **KOBAYASHI, Kei
Tokyo 130-8603 (JP)**
• **SASAKI, Keisuke
Tokyo 130-8603 (JP)**

(74) Representative: **Hoffmann Eitle
Patent- und Rechtsanwälte PartmbB
Arabellastraße 30
81925 München (DE)**

(54) **ORAL COMPOSITION**

(57) An oral composition containing nicotine, the bulk density of the oral composition being 0.4 g/cm$^3$ or greater.

**Description**

TECHNICAL FIELD

The present invention relates to an oral composition.

BACKGROUND ART

Oral compositions, which are fillings of oral pouch products, typically contain an organic material, such as microcrystalline cellulose (MCC), as a substrate. However, particles of such an organic substrate swell when exposed to moisture or heat, resulting in a decrease in the bulk density of the resulting composition after production. Therefore, the amount of composition necessary for ingesting a predetermined amount of nicotine must be inevitably increased, and it is necessary to use a large pouch with an unpleasant mouthfeel. PTL 1 discloses an elongated oral pouch product having a short side of 10 mm and a ratio of the long side to the short side in a range of from 3 to 6 for the purpose of fitting to the mouth of the user.

CITATION LIST

PATENT LITERATURE

PTL 1: European Patent Application Publication No. 3087852

SUMMARY OF INVENTION

TECHNICAL PROBLEM

The inventors have conceived that use of an oral composition having a specific bulk density enables a smaller oral pouch product to be provided. In view of the circumstances described above, an object of the present invention is to provide an oral composition capable of achieving a smaller oral pouch product.

SOLUTION TO PROBLEM

The inventors have found that the above problem can be solved by an oral composition having a specific bulk density.

Aspect 1

An oral composition containing nicotine, wherein the oral composition has a bulk density of 0.4 g/cm$^3$ or more.

Aspect 2

The oral composition according to aspect 1, containing an inorganic porous substrate.

Aspect 3

The oral composition according to aspect 2, wherein a content of the inorganic porous substrate is 6% by weight or more.

Aspect 4

The oral composition according to aspect 2 or 3,
wherein the inorganic porous material is selected from the group consisting of silica, silicate, alumina, zeolite, hydroxyapatite, hydrotalcite, and combinations thereof.

Aspect 5

The oral composition according to any one of aspects 2 to 4, wherein an average particle size of the inorganic porous substrate is 60 to 500 μm.

Aspect 6

The oral composition according to any one of aspects 1 to 5, having a water content of 10% to 70% by weight.

Aspect 7

An oral pouch product including:

   the oral composition according to any one of aspects 1 to 6; and
   a packaging material in which the oral composition is packaged.

ADVANTAGEOUS EFFECTS OF INVENTION

The present invention can provide an oral composition capable of achieving a smaller oral pouch product.

DESCRIPTION OF EMBODIMENTS

The present invention will be described in detail below. In the present invention, "X to Y" includes end values thereof, that is, X and Y. An oral product refers to a product whose active ingredient is ingested through the oral mucosa via saliva while the product is present in the oral cavity. An oral composition refers to a composition used in such an oral product.

1. Oral Composition

An oral composition according to the present embodiment (hereinafter, also simply referred to as a "composition") contains nicotine and preferably contains, as a substrate, an inorganic porous material (inorganic porous substrate). The substrate is a material that forms a matrix of the composition and is also referred to as an excipient.

(1) Bulk Density

The composition has a bulk density of 0.4 g/cm$^3$ or more. The composition having such a bulk density can provide a compact oral pouch product. The upper limit of the bulk density is not limited but is 0.8 g/cm$^3$ or less from the viewpoint of adjusting the amounts of components in the composition to appropriate ranges. The bulk density is measured in accordance with the Japanese Pharmacopoeia 3.01. Specifically, a composition powder is caused to fall freely through a hopper disposed at an upper position and caused to flow down to a 100 cm$^3$ measurement container until the powder overflows. The powder on the upper surface and the side surface of the measurement container is scraped off, and the weight of the composition powder is measured. An aerated bulk density is calculated by a formula below and determined as the bulk density.

$$\text{Aerated bulk density (g/cm}^3\text{)} = \text{weight of composition powder (g)/volume of measurement container (cm}^3\text{)}$$

)

(2) Nicotine

The composition contains nicotine. Nicotine may be contained in the form of nicotine alone or contained in the form of a nicotine-containing raw material. The nicotine-containing raw material refers to a raw material containing nicotine such as a nicotine salt or stabilized nicotine. An example of stabilized nicotine is a nicotine-carrying substance, such as nicotine supported on an ion-exchange resin. The ion-exchange resin may be a weakly acidic cation-exchange resin. The ion-exchange resin on which nicotine is supported can be specifically a resin complex containing, for example, 10% by weight or more and 20% by weight or less of nicotine, which is called nicotine polacrilex. The ion-exchange resin used in nicotine polacrilex is a weakly acidic cation-exchange resin. When nicotine polacrilex is used, the amount of nicotine polacrilex added relative to the oral composition is typically 0.5% by weight or more, preferably 10% by weight or more, more preferably 2.0% by weight or more. On the other hand, from the viewpoint of taste, the amount of nicotine polacrilex added relative to the composition is typically 15.0% by weight or less, preferably 12.0% by weight or less, more preferably 10.0% by weight or less.
The nicotine-containing raw material may be, for example, a tobacco material containing a tobacco powder obtained by grinding tobacco leaves. The tobacco powder may include, for example, shreds, a fine powder, or fibers of the lamina of dried tobacco leaves and is prepared by, for example, the method described later. Tobacco leaves may include mesophyll (lamina), leaf veins (stem), or roots. The tobacco material may include elements derived from midribs or roots of tobacco leaves, in addition to a tobacco powder basically obtained from the lamina of tobacco leaves.

The particle size of the tobacco powder is not limited. However, from the viewpoints of making fit in the oral cavity satisfactory to enhance the feeling of use, and satisfactorily releasing a flavor component contained in the tobacco powder into the oral cavity, the tobacco powder is preferably a powder that has passed through a 1.2 mm mesh, more preferably a powder that has passed through a 1.0 mm mesh.

Examples of the tobacco varieties serving as a raw material of the tobacco powder include, but are not particularly limited to, the genus *Nicotiana,* such as the flue-cured tobacco and burley tobacco of *Nicotiana tabacum* and the Brazilian tobacco of *Nicotiana rustica.* For the tobacco material and tobacco leaves described later, the same varieties as these can be used.

The tobacco powder is preferably prepared as follows. First, a base is added to and mixed with a tobacco powder obtained by grinding tobacco leaves. The base to be added is, for example, potassium carbonate or sodium carbonate, and the base is preferably add as an aqueous solution. A pH adjuster such as sodium dihydrogenphosphate may be added, for example, for the stabilization of nicotine during the production of an oral pouch product. The pH of the mixture after the addition of the base is preferably adjusted to 8.0 to 9.0. The tobacco powder content in this mixture is preferably 60% to 90% by weight.

After the addition of the base, heating is performed, for example, for 0.5 to 3 hours, preferably 0.8 to 2 hours under conditions under which, for example, the mixture temperature is 65°C to 90°C, preferably, the mixture temperature is 70°C to 80°C. The sterilization of the tobacco powder is thereby performed. The heating can be performed by either one or both of heating by steam injection and heating with a jacket. The pH of the mixture after the heating is preferably 8.0 to 9.0, and the moisture content of the mixture after the heating is preferably 10% to 50% by weight.

In an embodiment, after the heating, the resulting treated tobacco powder may be subjected to drying treatment by performing heating with a jacket alone, after steam injection is stopped as necessary. Subsequently, the tobacco powder may be cooled at about 15°C to 25°C for about one hour.

When a tobacco material containing a tobacco powder is used, the amount of tobacco material added relative to the oral composition is typically 0.001% by weight or more, preferably 0.01% by weight or more, more preferably 0.05% by weight or more. On the other hand, from the viewpoint of taste, the amount of tobacco material added relative to the composition is typically 90% by weight or less, preferably 80% by weight or less, 70% by weight or less, 45% by weight or less, 40% by weight or less, or 30% weight or less.

The nicotine-containing raw material may be a nicotine-containing extract liquid obtained through extraction from a nicotine-containing substance, such as tobacco leaves.

Of the embodiments described above, the use of a nicotine-carrying substance is preferred from the viewpoints of appropriate supply of nicotine and the ease of handling. In addition, when a tobacco powder is added, the color of the oral composition and the oral product usually tends to be the color of tobacco leaves. In contrast, when a colorless nicotine-containing compound is used, a white composition and a white oral product can be provided. For users who like white oral products, such an embodiment is advantageous. The raw materials described above may be used alone or in combination of two or more thereof.

The total nicotine content in the composition is typically, but not limited to, 0.1% to 5.0% by weight from the viewpoint of user preference. The total nicotine content in the composition is typically, 0.1% by weight or more, preferably 1.0% by weight or more, more preferably 2.0% by weight or more. On the other hand, from the viewpoint of taste, the total nicotine content in the composition is typically 5.0% by weight or less, preferably 4.0% by weight or less, more preferably 3.0% by weight or less. Accordingly, when a nicotine-containing raw material is used as a vegetable alkaloid, the amount of the raw material is adjusted such that the total nicotine content is within this range. When nicotine is present as ions, the above content is a content as nicotine ions. The nicotine content in the composition can be measured by, for example, gas chromatography-mass spectrometer (GC-MS) or liquid chromatography (LC, UV detection).

(3) Inorganic Porous Material

An inorganic porous material is used as a substrate. The inorganic porous material is preferably selected from the group consisting of silica, silicate, alumina, zeolite, hydroxyapatite, hydrotalcite, and combinations thereof. Of these, silica is preferred from the viewpoints of, for example, availability and little influence on flavor.

In the composition, the inorganic porous material content is preferably 6% by weight or more. The content refers to an amount in an absolute dry state unless otherwise specified. The lower limit value of the content is preferably 10% by weight or more, more preferably 15% by weight or more. The upper limit of the content is not limited but is typically 70% by weight or less, preferably 68% by weight or less, more preferably 65% by weight or less from the viewpoint of the limit to which other raw materials can be blended.

The inorganic porous material preferably has an average particle size of 60 to 500 $\mu$m. The average particle size of each material is a particle size at a cumulative volume of 50% (D50) in a particle size distribution determined by laser diffraction particle size distribution measurement. The laser diffraction particle size distribution measurement can be performed using, for example, Mastersizer 3000 (manufactured by Malvern Panalytical Ltd.). The average particle size means a particle size at a cumulative volume of 50% (D50) in a particle size distribution unless otherwise specified. When the average particle size is within the above range, the bulk density within the above range is likely to be achieved. If the average particle size is less than the lower limit value, for example, leakage of the powder from a pouch, which is a packaging material for the oral composition, and adhesion failure in sealing may be caused. This makes the user feel uncomfortable during use and reduces his/her preference. If the average particle size is excessively large, the user is likely to perceive the roughness due to particles of the inorganic porous material and may find the mouthfeel of the product unpleasant. From this viewpoint, the lower limit value of the average particle size is preferably 60 $\mu$m or more, more preferably 100 $\mu$m or more, and the upper limit value is preferably 500 $\mu$m or less, more preferably 300 $\mu$m or less.

(3) pH Adjuster

The composition contains a pH adjuster. The pH adjuster is not limited and is preferably one that is allowed to be added to food. Examples thereof include sodium carbonate, sodium hydrogencarbonate, potassium carbonate, potassium hydrogencarbonate, potassium phosphate, anhydrous sodium phosphate, sodium dihydrogenphosphate, and sodium citrate. Of these, sodium phosphate, potassium carbonate, or sodium dihydrogenphosphate is preferred from the viewpoints of influences on the taste of the product and product stability during storage. One of these pH adjusters may be used alone, or two or more of them may be used in combination in any ratio.

(4) Gelling Agent or Gelling Aid

The composition may contain a known gelling agent or the like. The gelling agent alleviates a feeling of a foreign body caused by a nonwoven fabric or the like when the product is taken into the oral cavity, in particular, at an initial point of time of taking it, and provides a favorable impression to the user. The gelling agent affects the bulk density of the composition. The gelling agent is preferably a polysaccharide having a carboxyl group as a functional group. For example, carrageenan, pectin, gum arabic, xanthan, gellan gum, and tragacanth gum are preferred. Furthermore, carrageenan, pectin, and gellan gum are preferred from the viewpoint that they are easily gelled in the presence of calcium ions, and that a carboxyl group and a cation can form a junction zone together to form a crosslinked structure. One of these may be used alone, or two or more of them may be used in combination in any ratio. In particular, from the viewpoint of achieving the bulk density within the above range, the gelling agent is preferably pectin or gellan gum.

A gelling aid component is, for example, calcium ions, and examples of the source thereof (gelling aids) include, but are not limited to, halogen acid salts (such as chloride), citric acid, carbonate, sulfate, phosphate, and lactate of calcium. Of these, calcium lactate, calcium chloride, or calcium phosphate is preferred, and calcium lactate is particularly preferred, from the viewpoints of little influence on the taste, high solubility, and the pH after dissolution. One of these may be used alone or in combination of two or more thereof in any ratio.

Examples of the gelling aid component other than calcium ions include ions of metals such as magnesium, silver, zinc, copper, gold, and aluminum, with which the gelling agent can be bound through ionic bonds as with calcium ions, and ions of cationic polymers. Examples of the source thereof (other gelling aids) include halogen acid salts (such as chloride), citric acid, carbonates, sulfates, and phosphates of these metal ions and cationic polymers. One of these may be used alone, or two or more of them may be used in combination in any ratio.

(5) Release Agent

The composition may contain a known release agent. However, since the inorganic porous material, in particular, silica also has the function as a release agent, a release agent other than the inorganic porous material may not be contained.

(6) Water

The water content (moisture content) in the composition is 10% to 70% by weight in view of the ease of production of the composition. Furthermore, from the viewpoints of improving production efficiency of the composition, improving caking resistance of the composition, and reducing the stickiness of the composition, the lower limit of the water content is preferably 30% by weight or more, more preferably 45% by weight or more, and the upper limit is preferably 60% by weight or less, more preferably 50% by weight or less. The water content may be 40% by weight or less, may be 30% by weight or less, or may be 20% by weight or less. The water content can be controlled by adjusting the amount of water added or performing heating treatment or drying treatment during the production stage. The water content of the composition is controlled as appropriate according to the type of product (moist or dry). In the case of the moist type, for example, the water content is typically 20% to 60% by weight, preferably 30% to 50% by weight. On the other hand, in the case of the dry type, the water content is typically 5% to 20% by weight, preferably 10% to 15% by weight.

The water content (moisture content) of the composition can be measured with a heat-drying moisture meter (for example, HB 43-S: manufactured by METTLER TOLEDO). In the measurement, a sample is put in a predetermined container and heated to an attained temperature of 100°C. The measurement is terminated at the time when the amount of change becomes 1 mg or less per 60 seconds, and the moisture content is calculated from the weighed values before and after heating.

(7) Others

The composition may contain other substances besides the foregoing. Examples of the other substances include a flavoring agent, a sweetener, a humectant, a bitterness inhibitor, and an emulsifier. The contents of the substances are not limited, and their formulation can be appropriately adjusted according to the product design.

1) Flavoring Agent

The flavoring agent is not limited, and examples thereof include menthol, leaf tobacco extract, natural vegetable flavoring agents (e.g., cinnamon, sage, herb, chamomile, kudzu, sweet Hydrangea leaves, clove, lavender, cardamom, Eugenia caryophyllus, nutmeg, bergamot, geranium, honey essence, rose oil, lemon, orange, cassia bark, caraway, jasmine, ginger, coriander, vanilla extract, spearmint, peppermint, cassia, coffee, celery, cascarilla, sandalwood, cocoa, ylang-ylang, fennel, anise, licorice, Saint John's bread, plum extract, and peach extract), saccharides (e.g., glucose, fructose, isomerized sugar, caramel, honey, and molasses), cocoas (e.g., powder and extract), esters (e.g., isoamyl acetate, linalyl acetate, isoamyl propionate, and linalyl butyrate), ketones (e.g., menthone, ionone, damascenones, and ethyl maltol), alcohols (e.g., geraniol, linalool, anethole, and eugenol), aldehydes (e.g., vanillin, benzaldehyde, and anisaldehyde), lactones (e.g., $\gamma$-undecalactone and $\gamma$-nonalactone), animal flavoring agents (e.g., musk, ambergris, civet, and castoreum), and hydrocarbons (e.g., limonene and pinene). One of these flavoring agents may be used alone, or two or more of them may be used in combination in any ratio.

2) Sweetener

Examples of the sweetener include, but are not limited to, sugar alcohols such as xylitol, maltitol, and erythritol; and acesulfame potassium, sucralose, and aspartame. From the viewpoint of taste adjustment, sugar alcohols are preferred. One of these sweeteners may be used alone, or two or more of them may be used in combination in any ratio.
The type of sugar alcohol is not particularly limited, and examples thereof include xylitol, maltitol, erythritol, sorbitol, mannitol, and lactitol. Of these, maltitol is preferred from the viewpoint of imparting a good taste. One of these substances may be used alone, or two or more of them may be used in combination in any ratio.
The sugar alcohol content in the composition (when the composition contains two or more sugar alcohols, their total content) is not limited. However, from the viewpoint of taste adjustment, the sugar alcohol content is typically 1% by weight or more, preferably 5% by weight or more, more preferably 10% by weight or more. The upper limit is typically 80% by weight or less, preferably 70% by weight or less, more preferably 60% by weight or less.

3) Bitterness Inhibitor

The bitterness inhibitor is not limited, but an example thereof is soybean lecithin. Soybean lecithin is a phospholipid, and examples thereof include phosphatidylcholine, phosphatidylethanolamine, and phosphatidic acid. One of these bitterness inhibitors may be used alone, or two or more of them may be used in combination in any ratio.

4) Humectant

The humectant is not limited, but examples thereof include polyhydric alcohols, such as glycerine and propylene glycol. From the viewpoint of product storage stability, glycerine is preferred. One of these humectants may be used alone, or two or more of them may be used in combination in any ratio.

5) Emulsifier or Surfactant

The emulsifier is are not limited, but examples thereof include emulsifiers that are added to food. The emulsifier may be one or more selected from the group consisting of sucrose fatty acid esters, organic acid glycerine fatty acid esters, polyglycerine fatty acid esters, and lecithins. Examples of sucrose fatty acid esters include sucrose palmitate and sucrose stearate. Examples of organic acid glycerine fatty acid esters include succinic acid glycerine fatty acid esters and diacetyltartaric acid glycerine fatty acid esters. Examples of polyglycerine fatty acid esters include decaglycerine fatty acid esters. The emulsifier content in the composition is preferably an amount such that the total content including the above-described polyglycerine fatty acid ester falls within the above ranges described as the contents of the polyglycerine fatty acid esters.

The degree of polymerization of glycerine in a polyglycerine fatty acid ester is preferably 2 to 10. The polyglycerine fatty acid ester functions as an emulsifier. Thus, incorporating a polyglycerine fatty acid ester holds a state in which the components in the composition are homogeneously mixed and can stably keep the flavor component; therefore, a good flavor of the composition can be provided. In addition, the polyglycerine fatty acid ester can impart a moderate viscosity to the composition to allow the components to be bound together, and thus can suppress dryness of the composition to make the feeling of use, flavor, and the like good. In addition, even in the case where the composition is of dry type, which has a low water content (moisture content), it is possible to reduce the scattering of the composition when the composition is packed in an exterior material such as a pouch. Thus, incorporating a polyglycerine fatty acid ester in the composition enables improvements in production efficiency in the production of the oral product, such as the operating efficiency and the yield.

Polyglycerine fatty acid esters are fatty acid esters of a dehydrated condensate of glycerine. The degree of polymerization of the glycerine is typically 2 or more and may be 3 or more, and is typically 10 or less and may be 8 or less.

Fatty acid ester groups (RCOO- groups) in a polyglycerine fatty acid ester are derived from a fatty acid. The fatty acid is not limited, and may be a saturated fatty acid or may be an unsaturated fatty acid. From the viewpoints of a good flavor and production efficiency, the number of carbon atoms in the fatty acid is typically 10 or more, preferably 12 or more, more preferably 14 or more, still more preferably 16 or more, and the number of carbon atoms is typically 30 or less, preferably 26 or less, more preferably 22 or less, still more preferably 20 or less. The fatty acid may have a substituent or may be unsubstituted.

The number of fatty acid ester groups that one molecule of the polyglycerine fatty acid ester has is not limited as long as the polyglycerine fatty acid ester has a structure with which it can exert the function as an emulsifier and can be appropriately selected according to the degree of polymerization of glycerine and the number of hydroxyl groups derived from glycerine. The structure with which the polyglycerine fatty acid ester can exert the function as an emulsifier is a structure having both a fatty acid moiety, which serves as a lipophilic group, and a polyhydric alcohol moiety, which serves as a hydrophilic group. Specifically, it is only necessary that the number of fatty acid ester groups per molecule of the polyglycerine fatty acid ester be typically 1 or more. It is only necessary that the number of hydroxyl groups derived from glycerine be 1 or more.

The degree of polymerization of glycerine and the type and number of fatty acid ester groups in the polyglycerine fatty acid ester can be any combination of those described above. More specifically, the alcohol component of the polyglycerine fatty acid ester may be diglycerine, triglycerine, tetraglycerine, pentaglycerine, hexaglycerine, heptaglycerine, octaglycerine, nonaglycerine, or decaglycerine. The acid component of the polyglycerine fatty acid ester may be a fatty acid such as lauric acid, myristic acid, palmitic acid, stearic acid, oleic acid, linoleic acid, or $\alpha$-linolenic acid. The polyglycerine fatty acid ester may be a monoester, a diester, a triester, a tetraester, a pentaester, or the like. One of these polyglycerine fatty acid esters may be used alone, or two or more of them may be used in combination in any ratio.

From the viewpoints of a good flavor and the workability during production, the polyglycerine fatty acid ester is preferably one or more selected from diglycerine monofatty acid esters and decaglycerine fatty acid esters. A diglycerine monofatty acid ester is preferably selected from the group consisting of diglycerine monolaurate, diglycerine monomyristate, diglycerine monopalmitate, diglycerine monostearate, and diglycerine monooleate and is more preferably diglycerine monooleate. A decaglycerine fatty acid ester is preferably selected from the group consisting of decaglycerine laurates, decaglycerine myristates, decaglycerine palmitates, decaglycerine stearates, and decaglycerine oleates and is more preferably selected from the group consisting of decaglycerine monolaurate, decaglycerine monomyristate, decaglycerine monopalmitate, decaglycerine monostearate, and decaglycerine monooleate.

The polyglycerine fatty acid ester content in the composition (when the composition contains two or more polyglycerine fatty acid esters, their total content) is not limited; however, from the viewpoints of obtaining a good flavor and improving production efficiency, the content is typically 0.1% by weight or more, preferably 0.2% by weight or more, more preferably 0.3% by weight or more, still more preferably 0.5% by weight or more. From the viewpoint of imparting a moderate viscosity to the composition, the polyglycerine fatty acid ester content is typically 20.0% by weight or less, preferably 15.0% by weight or less, more preferably 10.0% by weight or less, still more preferably 8.0% by weight or less.

The HLB value of the polyglycerine fatty acid ester is not limited. However, from the viewpoints of obtaining a good flavor and improving production efficiency, the HLB value is typically 6.0 or more, preferably 7.0 or more, and is typically 20.0 or less, preferably 18.0 or less, more preferably 16.0 or less.

(8) Property of Composition

1) pH

The pH of the composition is not limited. However, in view of the influence on the taste, the pH is typically 7.0 or more, preferably 7.5 or more, more preferably 8.0 or more, and is typically 10.0 or less, preferably 9.5 or less, more preferably 9.0 or less. The pH is a value measured at 25°C.

The pH of the composition at a measurement temperature of 25°C can be measured with a pH analyzer (for example, LAQUA F-72 with a flat ISFET pH electrode: manufactured by HORIBA, Ltd.) by adding 20 mL of water relative to 2 g of the composition, shaking the mixture for 10 minutes, and analyzing the supernatant. The calibration of the device is preferably conducted by, for example, three-point calibration using a phthalate pH standard solution (pH 4.01), a phosphate pH standard equimolal solution (pH 6.86), and a tetraborate pH standard solution (pH 9.18) (all available from FUJIFILM Wako Pure Chemical Corporation).

(9) Method for Producing Composition

The composition is produced by any method, but is preferably produced through a step of mixing nicotine, and optionally a substrate and the components described above. The mixing can be performed by putting all raw materials into a mixer and mixing the raw materials.

In a preferred production method, the substrate, nicotine, and optionally water and other substances (such as a sweetener, a flavoring agent, and a humectant) are first mixed together to prepare a first mixture. During this mixing, heating may be performed. The order of mixing of the raw materials is not limited; the raw materials may be put into the mixer and mixed together in any order or simultaneously, or solid raw materials may be homogeneously mixed, liquid raw materials may then be added to the mixture, and mixing may be further performed. In view of workability, the latter embodiment is preferred.

To the mixture obtained as described above, an aqueous solution containing a pH adjuster, a sweetener such as acesulfame potassium, a flavoring agent such as menthol, a bitterness inhibitor such as soybean lecithin, and a humectant such as glycerine can optionally be added (additive addition step). The above additives may be added in the form of solids or may be added in the form of aqueous solutions in which the additives are dissolved in water. When the additives are added in the form of aqueous solutions, the additives may be dissolved in advance in predetermined amounts of water and added so that the final water content of the oral product can be achieved.

2. Oral Pouch Product

The oral product is used by taking the product in the mouth. An oral pouch product refers to a product including a sealed water-insoluble packaging material (also referred to as a pouch) and a composition (also referred to as a main material or filling) contained in the packaging material, in which saliva permeates through the pouch and dissolves components in the composition contained in the pouch, and the components can subsequently pass through the pouch and be carried into the oral cavity.

(1) Pouch

The pouch is not limited and a known one can be used as long as the pouch enables the filling to be packaged therein, is insoluble in water, and is permeable to liquids (such as water and saliva) and water-soluble components in the filling. An example of the material of the pouch is a cellulose-based nonwoven fabric, and a commercially available nonwoven fabric may be used. A pouch product can be produced by forming a sheet made of such a material into a bag shape, packing a filling into the bag, and sealing the bag by means of heat sealing or the like.

The basis weight of the sheet is not particularly limited and is typically 12 gsm ($g/m^2$) or more and 54 gsm or less, preferably 24 gsm or more and 30 gsm or less. The thickness of the sheet is not particularly limited and is typically 100 $\mu$m or more and 300 $\mu$m or less, preferably 175 $\mu$m or more and 215 $\mu$m or less.

At least one of the inner surface and outer surface of the pouch may be partially coated with a water-repellent material. A water-repellent fluororesin is suitable for the water-repellent material. Specifically, an example of a water-repellent fluororesin of this type is AsahiGuard (registered trademark) manufactured by AGC Inc. Water-repellent fluororesins are applied to packaging materials for fat-containing foods and products, such as confectionery, dairy products, ready-prepared foods, fast foods, and pet foods. Accordingly, water-repellent fluororesins of this type are safe even when applied to a pouch to be placed in the oral cavity. This water-repellent material is not limited to a fluororesin and may be a material having a water-repellent effect, such as a paraffin resin, a silicone resin, or an epoxy resin.

The pouch may contain optional components. Examples of such components include raw materials for adjusting flavor and taste, flavoring agents, additives, tobacco extract liquids, and coloring agents. These components may be incorporated in any form. For example, the components may be applied to or infiltrated into the surface of the pouch or, when the pouch is composed of fibers, the components may be incorporated into the fibers.

The appearance of the pouch is also not limited. The pouch may be nontransparent, translucent, or transparent. When the pouch is translucent or transparent, the filling can be seen through.

As for the size of the oral pouch product before use, the upper limit of the long side is preferably 25 mm or less, more preferably 15 mm or less. The lower limit of the short side is preferably 10 mm or more or 13 mm or more, and the upper limit is preferably 20 mm or less or 17 mm or less. The lengths of the long side and the short side are lengths that do not include edge portions of the pouch. The ratio of the weight of the filling to the total weight of the oral pouch product is not limited, but is typically 80% by weight or more, preferably 85% by weight or more, more preferably 90% by weight or more. The ratio is typically 99% by weight or less, preferably 97% by weight or less, more preferably 95% by weight or less.

(2) Filling

The oral pouch product is filled with the above-described composition as a filling. The amount of filling per oral pouch product is preferably 0.4 to 1.5 g.

(3) Method for Producing Oral Pouch Product

The oral pouch product can be produced by packaging the composition with an exterior material (packaging step). The packaging method is not limited, and a known method can be applied. For example, a known method can be employed in which the composition is put into a bag-shaped nonwoven fabric, followed by sealing. In the packaging step, desired water may be further added after sealing (water addition step). For example, when the water content of the final composition is 50% by weight and the water content in the packed composition is 15% by weight, water equivalent to the remaining 35% by weight is added.

Aspects will be described below.

Aspect 1

An oral composition containing nicotine, wherein the oral composition has a bulk density of 0.4 g/cm$^3$ or more.

Aspect 2

The oral composition according to aspect 1, containing an inorganic porous substrate.

Aspect 3

The oral composition according to aspect 2, wherein a content of the inorganic porous substrate is 6% by weight or more.

Aspect 4

The oral composition according to aspect 2 or 3, wherein the inorganic porous material is selected from the group consisting of silica, silicate, alumina, zeolite, hydroxyapatite, hydrotalcite, and combinations thereof.

Aspect 5

The oral composition according to any one of aspects 2 to 4, wherein an average particle size of the inorganic porous substrate is 60 to 500 $\mu$m.

Aspect 6

The oral composition according to any one of aspects 1 to 5, having a water content of 10% to 70% by weight.

Aspect 7

An oral pouch product including:

the oral composition according to any one of aspects 1 to 6; and
a packaging material in which the oral composition is packaged.

EXAMPLES

[Example 1] Production of Oral Composition

Silica (SIPERNAT 2200, manufactured by Evonik Industries) (100.0 g) serving as a substrate, 3.87 g of nicotine, 45.1 g of an aqueous solution of anhydrous sodium phosphate, 4.79 g of an aqueous solution of anhydrous citric acid, 14.03 g of an aqueous solution of sodium chloride, and 23.4 g of an aqueous solution of acesulfame K were added, and these were mixed until homogeneous to obtain a mixture A. The obtained mixture A was subjected to jacket heating (can wall temperature: 100°C). The mixture was then cooled for 30 minutes under the condition of an ambient temperature of 20°C to obtain a mixture B (moisture after cooling: 11%). To the mixture B after cooling, 67.7 g of an aqueous solution of tripotassium phosphate, 28.5 g of an aqueous solution of trisodium phosphate, 20 g of an aqueous solution of gellan gum, 7.5 g of a flavoring agent, and other components were added to obtain 270 g of an oral composition (moisture content 44.06% by weight, pH 8.46).

[Example 2]

An oral composition was produced in the same manner as in Example 1 except that 7.5 g of distilled water was mixed instead of the flavoring agent.

[Example 3]

An oral composition was produced in the same manner as in Example 1 except that 25.0 g of powdered cellulose (VITACEL L00, manufactured by J. Rettenmaier & Söhne) and 75.0 g of the silica were mixed and used as a substrate.

[Example 4]

An oral composition was produced in the same manner as in Example 1 except that 35.0 g of the powdered cellulose and 66.0 g of the silica were mixed and used as a substrate.

[Example 5]

An oral composition was produced in the same manner as in Example 1 except that 50.0 g of the powdered cellulose and 51.0 g of the silica were mixed and used as a substrate.

[Example 6]

An oral composition was produced in the same manner as in Example 1 except that 76.0 g of the powdered cellulose and 26.0 g of the silica were mixed and used as a substrate.

[Comparative Example 1]

An oral composition was produced in the same manner as in Example 1 except that 100.0 g of granular regenerated cellulose (Viscopearl Mini, manufactured by Rengo Co., Ltd.) was used as a substrate.

[Comparative Example 2]

An oral composition was produced in the same manner as in Example 1 except that 100.0 g of the granular regenerated cellulose was used as a substrate, and 7.5 g of distilled water was added instead of the flavoring agent.

[Comparative Example 3]

An oral composition was produced in the same manner as in Example 1 except that 81.0 g of the powdered cellulose and 20.0 g of the silica were mixed and used as a substrate. Table 1 shows the compositions and physical properties of the compositions.

[Table 1]

| | | Example | | | | | | Comparative Example | | |
|---|---|---|---|---|---|---|---|---|---|---|
| | | 1 | 2 | 3 | 4 | 5 | 6 | 1 | 2 | 3 |
| Amount blended % by weight | Nicotine | 1.37 | 1.37 | 1.37 | 1.37 | 1.37 | 1.37 | 1.37 | 1.37 | 1.37 |
| | Substrate (Total) | 33.14 | 33.14 | 33.14 | 33.14 | 33.14 | 33.14 | 33.14 | 33.14 | 33.14 |
| | - Cellulose in substrate | - | - | 8.29 | 11.6 | 16.57 | 24.89 | 33.14 | 33.14 | 26.51 |
| | - Silica in substrate | 33.14 | 33.14 | 24.86 | 21.54 | 16.57 | 8.29 | - | - | 6.63 |
| | Tobacco | 0.02 | 0.02 | 0.02 | 0.02 | 0.02 | 0.02 | 0.02 | 0.02 | 0.02 |
| | Citric acid | 1.52 | 1.52 | 1.52 | 1.52 | 1.52 | 1.52 | 1.52 | 1.52 | 1.52 |
| | $NaH_2PO_4$ | 4.23 | 4.23 | 4.23 | 4.23 | 4.23 | 4.23 | 4.23 | 4.23 | 4.23 |
| | $K_3PO_4$ | 11.44 | 11.44 | 11.44 | 11.44 | 11.44 | 11.44 | 11.44 | 11.44 | 11.44 |
| | $Na_3PO_4$ | 1.25 | 1.25 | 1.25 | 1.25 | 1.25 | 1.25 | 1.25 | 1.25 | 1.25 |
| | NaCl | 1.33 | 1.33 | 1.33 | 1.33 | 1.33 | 1.33 | 1.33 | 1.33 | 1.33 |
| | Acesulfame K | 1.66 | 1.66 | 1.66 | 1.66 | 1.66 | 1.66 | 1.66 | 1.66 | 1.66 |
| | Gellan gum | 0.05 | 0.05 | 0.05 | 0.05 | 0.05 | 0.05 | 0.05 | 0.05 | 0.05 |
| | Flavoring agent | 0 | 0 | 2.98 | 2.98 | 2.98 | 2.98 | 2.98 | 0 | 2.98 |
| | Water | 41 | 44 | 41 | 41 | 41 | 41 | 41 | 44 | 41 |
| | Total | 100 | 100 | 100 | 100 | 100 | 100 | 100 | 100 | 100 |
| Water content (% by weight) | | 40.08 | 44.06 | 41.2 | 41.2 | 41.2 | 41.2 | 41.12 | 45.12 | 41.2 |
| pH | | 8.58 | 8.46 | 8.51 | 8.51 | 8.51 | 8.51 | 8.5 | 8.5 | 8.51 |
| Bulk density (g/cm$^3$) | | 0.7051 | 0.7436 | 0.5378 | 0.4936 | 0.4493 | 0.4027 | 0.3165 | 0.3284 | 0.3634 |

[Example 7]

An oral product was produced by collecting 0.4 g of the composition obtained in Example 1 and packaging the composition in a known pouch.

[Example 8]

An oral product was produced by collecting 1.0 g of the composition obtained in Example 1 and packaging the composition in a known pouch.

[Comparative Example 4]

An oral product was produced by collecting 0.4 g of the composition obtained in Example 1 and packaging the composition in a known pouch.

EP 4 516 122 A1

[Comparative Example 5]

An oral product was produced by collecting 0.65 g of the composition obtained in Example 1 and packaging the composition in a known pouch. Table 2 shows the sizes of the produced oral products.

[Table 2]

|  | Example 7 | Example 8 | Comparative Example 4 | Comparative Example 5 |
|---|---|---|---|---|
| Substrate material | Silica | Silica | Regenerated cellulose | Regenerated cellulose |
| Long side (mm) | 22 | 10 | 31 | 26 |
| Long side (including sealing portions) (mm) | 26 | 14 | 35 | 30 |
| Short side (mm) | 14 | 14 | 14 | 14 |
| Long-short ratio | 1.9 | 1.0 | 2.5 | 2.1 |
| Filling (g) | 1.0 | 0.4 | 0.65 | 0.4 |
| Sealing portions (Total of both ends) (mm) | 4 | 4 | 4 | 4 |

Example 7, Example 8, Comparative Example 4, and Comparative Example 5 above were subjected to sensory evaluation by seven expert panelists. Since the oral products of Example 7 and Example 8 easily fitted into the space in the oral cavity, the panelists were less likely to have a feeling of a foreign body and tended to have high preference to the oral products. In contrast, since the products of Comparative Example 4 and Comparative Example 5 were large, when the panelists used the products in the space in their oral cavity, they tended to have low preference to the products compared with those of Example 7 and Example 8.

According to the composition of the present embodiment, a product that contains a small amount of the composition and achieves a sufficient flavor effect can be designed by controlling the bulk density of the composition. In particular, in a pouch-type oral product, when the size of the product is large, the user is likely to have a feeling of a foreign body caused by a nonwoven fabric of the packaging material, which may cause a reduction in his/her preference. That is, the oral composition according to the present embodiment is useful in that the degree of freedom in product design can be enhanced, and a product that does not interfere with the preference of the user can be provided. The physical properties were measured as follows.

<Aerated Bulk Density>

The aerated bulk density was measured in accordance with the Japanese Pharmacopoeia 3.01. Specifically, a composition powder of each of the examples was caused to fall freely through a hopper disposed at an upper position and caused to flow down to a 100 cm$^3$ measurement container until the powder overflowed. The powder on the upper surface and the side surface of the measurement container was scraped off, and the mass of the composition powder was measured. The aerated bulk density was determined by a formula below on the basis of the measured mass of the powder.

Aerated bulk density (g/cm$^3$) = mass of composition powder (g)/volume of measurement container (cm$^3$)

<Water Content>

The measurement was conducted with a heat-drying moisture meter (HB 43-S: manufactured by METTLER TOLEDO). In the measurement, a sample was put in a predetermined container and heated to an attained temperature of 100°C. The measurement was terminated at the time when the amount of change became 1 mg or less per 60 seconds, and the moisture content was calculated from the weighed values before and after heating.

<pH>

The measurement was conducted with a pH analyzer (LAQUA F-72 with a flat ISFET pH electrode: manufactured by HORIBA, Ltd.) by adding 20 mL of water relative to 2 g of a composition, shaking the mixture for 10 minutes, and analyzing the supernatant. The calibration of the device was conducted by, for example, three-point calibration using a phthalate pH standard solution (pH 4.01), a phosphate pH standard equimolal solution (pH 6.86), and a tetraborate pH standard solution (pH 9.18) (all available from FUJIFILM Wako Pure Chemical Corporation).

**Claims**

1.  An oral composition comprising nicotine, wherein the oral composition has a bulk density of 0.4 g/cm$^3$ or more.

2.  The oral composition according to claim 1, comprising an inorganic porous substrate.

3.  The oral composition according to claim 2, wherein a content of the inorganic porous substrate is 6% by weight or more.

4.  The oral composition according to claim 2 or 3,
    wherein the inorganic porous material is selected from the group consisting of silica, silicate, alumina, zeolite, hydroxyapatite, hydrotalcite, and combinations thereof.

5.  The oral composition according to any one of claims 2 to 4, wherein an average particle size of the inorganic porous substrate is 60 to 500 $\mu$m.

6.  The oral composition according to any one of claims 1 to 5, having a water content of 10% to 70% by weight.

7.  An oral pouch product comprising:

    the oral composition according to any one of claims 1 to 6; and
    a packaging material in which the oral composition is packaged.

# EP 4 516 122 A1

<table>
<tr><th colspan="2">INTERNATIONAL SEARCH REPORT</th><th>International application No.</th></tr>
<tr><td colspan="2"></td><td>PCT/JP2023/016862</td></tr>
</table>

**A. CLASSIFICATION OF SUBJECT MATTER**

*A24B 13/00*(2006.01)i
FI:   A24B13/00

According to International Patent Classification (IPC) or to both national classification and IPC

**B. FIELDS SEARCHED**

Minimum documentation searched (classification system followed by classification symbols)

A24B13/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2020/244722 A1 (NCP NEXTGEN A/S) 10 December 2020 (2020-12-10) page 2, lines 2-6, page 3, lines 1-3, page 4, lines 16-21 | 1-4, 6-7 |
| Y | | 4-7 |
| Y | JP 2017-536129 A (R. J. REYNOLDS TOBACCO CO.) 07 December 2017 (2017-12-07) paragraph [0096] | 4-7 |
| Y | JP 2014-506468 A (SURFLAY NANOTEC GMBH) 17 March 2014 (2014-03-17) paragraph [0033] | 5-7 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **29 May 2023** | **20 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/016862**

| Patent document cited in search report | | | Publication date (day/month/year) | Patent family member(s) | | | Publication date (day/month/year) |
|---|---|---|---|---|---|---|---|
| WO | 2020/244722 | A1 | 10 December 2020 | JP | 2022-535586 | A | |
| | | | | paragraphs [0005], [0008], [0016] | | | |
| | | | | US | 2021/0307375 | A1 | |
| | | | | EP | 3773495 | A1 | |
| | | | | CN | 113924004 | A | |
| | | | | KR | 10-2022-0019277 | A | |
| JP | 2017-536129 | A | 07 December 2017 | US | 2016/0157515 | A1 | |
| | | | | paragraph [0080] | | | |
| | | | | WO | 2016/090075 | A1 | |
| | | | | EP | 3226700 | A1 | |
| | | | | CN | 107205471 | A | |
| JP | 2014-506468 | A | 17 March 2014 | US | 2014/0246033 | A1 | |
| | | | | paragraph [0033] | | | |
| | | | | WO | 2012/110258 | A2 | |
| | | | | EP | 2675305 | A2 | |

Form PCT/ISA/210 (patent family annex) (January 2015)

**EP 4 516 122 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3087852 A